# EUROPÄISCHE PATENTSCHRIFT

(11) **EP 4 197 509 B1**
(45) Veröffentlichungstag und Bekanntmachung des Hinweises auf die Patenterteilung: **28.01.2026**
(21) Anmeldenummer: 22210715.3
(22) Anmeldetag: 01.12.2022
(51) Int. Cl.: A61F 13/551, A61F 13/84

(54) **HYGIENEARTIKEL**
SANITARY ARTICLE
ARTICLE D'HYGIÈNE PERSONNELLE

(30) Priorität: 15.12.2021 DE 102021133326
(43) Veröffentlichungstag der Anmeldung: 21.06.2023
(73) Patentinhaber: Paul Hartmann AG, 89522 Heidenheim (DE)
(72) Erfinder: BÄHRLE, Christian, 89542 Herbrechtingen (DE); HERZOG, Moritz, 73207 Plochingen (DE); HORN, Annette, 89231 Neu-Ulm (DE)
(74) Vertreter: DREISS Patentanwälte PartG mbB

(56) Entgegenhaltungen:
- WO-A1-2019/176961
- JP-B2- 4 518 325
- JP-B2- 5 946 695
- JP-U- 3 215 203
- US-A1- 2001 056 270

## Beschreibung

Die Erfindung betrifft einen einzeln verpackten Hygieneartikel, umfassend einen absorbierenden Artikel und ein den Artikel vorzugsweise vollständig umschließendes und von der Umgebung vorzugsweise weitestgehend abschließendes Verpackungsmittel, wobei der absorbierende Artikel zumindest ein körperzugewandtes Topsheet, ein körperabgewandtes Backsheet und einen dazwischen angeordneten Absorptionskörper aufweist, wobei eine körperabgewandte Außenseite des Backsheets bereichsweise eine Haftmittelbeschichtung zur lösbaren Anhaftung des absorbierenden Artikels an einem Bekleidungsstück aufweist. Bei hier in Rede stehenden absorbierenden Artikeln wird unter einem körperzugewandten Topsheet eine körperzugewandte flüssigkeitsdurchlässige Lage, zumeist auf Vlies- oder Filmbasis, und unter einem körperabgewandten Backsheet eine weitestgehend flüssigkeitsundurchlässige, insbesondere gleichwohl atmungsaktive, also wasserdampfdurchlässige Lage, zumeist auf Filmbasis verstanden.

Bei den einzeln umverpackten absorbierenden Artikeln kann es sich typischerweise um Damenhygieneartikel, aber beispielsweise auch um absorbierende Inkontinenzartikel handeln. Solche Artikel werden heutzutage optisch ansprechend gestaltet, und es wird häufig auf die Verwendung einer ein- oder mehrfarbigen Bedruckung hierfür zurückgegriffen. Hierbei ist es möglich, visuell wahrnehmbare Kennzeichnungs- oder Dekorationsmittel auf das Produkt selbst, etwa auf dessen Backsheet, oder auf die Verpackung des Produkts, insbesondere auf eine Einzelumverpackung des Produkts, aufzubringen.

WO 2021/153377 A1 offenbart einen einzeln umverpackten Hygieneartikel in Form einer Damenbinde mit einer die Haftmittelbeschichtung auf der Außenseite des Backsheets überfangenden Abdecklage. Diese Abdecklage umfasst zumindest auf ihrer dem Backsheet zugewandten Seite eine Antihafteigenschaft und dient dazu, ein Verkleben der Haftmittelbeschichtung mit der äußeren Verpackung zu verhindern. Nach der Entnahme aus der äußeren Verpackung wird dieser Haftmittelstreifen in an sich bekannter Weise abgelöst, so dass der absorbierende Artikel am Schrittsteg eines Bekleidungsstücks angehaftet werden kann. Diese Abdecklage ist dekorativ gestaltet und hat jedenfalls ein Dekorationsmittel, welches einen dekorativen Bereich bildet. Dieser dekorative Bereich der Abdecklage ist so angeordnet, dass er der Verpackungsschicht zugewandt ist und auch überlappend mit einem Fügebereich zwischen der Abdecklage und der äußeren Verpackungsschicht angeordnet ist, um diesen zu kaschieren. Die äußere Verpackung ist von einem Vlies oder Kunststofffilm gebildet.

WO 2013/080757 A1 offenbart ebenfalls einen einzeln verpackten Hygieneartikel, dessen die Verpackung bildendes Verpackungsmittel von einem mehrschichtigen Vliesmaterial gebildet ist. Das mehrschichtige Vliesmaterial umfasst eine innen liegende Meltblown-Vliesschicht und eine daran außen angrenzende Spunbond-Vliesschicht. Da Meltblown-Vliesschichten prozessbedingt sehr viel dünnere Fasern und typischerweise eine höhere Dichte als Spunbond-Vliesschichten aufweisen, eignen sie sich besser zum Bedrucken, da die Druckfarbe in die kapillaren Faserzwischenräume eintreten kann. Daher ist eine dekorative Bedruckung auf der dem Artikel zugewandten inneren Seite der aus Vlies bestehenden Verpackung aufgebracht. Dadurch, dass die äußere Spunbond-Vliesschicht längere und dickere Fasern und eine geringere Dichte aufweist, ist sie hinreichend lichtdurchlässig und nicht zu dicht, damit die Bedruckung auf der inneren Vliesschicht visuell wahrnehmbar ist. Auch scheint eine gewisse diffuse Lichtstreuung durch das Vliesmaterial erwünscht zu sein, damit der in der Verpackung aufgenommene Hygieneartikel nicht sofort als solcher erkennbar ist.

JP 4518325 B2 offenbart einen einzeln umverpackten Hygieneartikel mit einer Mehrzahl von verschiedenen Ausführungsvarianten mit und ohne Releaseschicht für die Abdeckung der Haftmittelbeschichtung auf dem Backsheet des Hygieneartikels, wobei Kennzeichnungs- oder Dekorationsmittel bei den verschiedenen Ausführungsvarianten bei dem Backsheet des Hygieneartikels selbst und/oder bei der Releaseschicht und/oder bei der Umverpackung vorgesehen sind.

Ein einzeln umverpackter Hygieneartikel mit den Merkmalen des Oberbegriffs des Anspruchs 1 ist vorbekannt aus WO 2019/176961.

Die Anmelderin hat festgestellt, dass es bei in herkömmlicher Weise auf ihrer Außenseite bedruckten Verpackungsmitteln gerade bei einzeln verpackten Hygieneartikeln zu Problemen kommen kann. So kann es im Zuge der Herstellung einzeln verpackter Hygieneartikel zu Relativbewegungen einzeln verpackter Hygieneartikel gegen Förderbänder oder Führungsschienen eines Transportwegs kommen, wodurch der Aufdruck abgerieben werden kann. Häufig werden einzeln verpackte Hygieneartikel auch zu Produktstapeln zusammengebracht und komprimiert, um sie in eine weitere Umverpackung für die Lagerhaltung und den Transport und den Verkauf zu bringen. Bei der Handhabung solcher komprimierter Produktstapel kommt es auf der Außenseite besagter Stapel aufgrund von Rückstellkräften der komprimierten Produkte zu signifikanten Reibungskräften zu Handhabungskomponenten von Verpackungseinheiten, aber auch zu Reibungskräften zwischen den Außenseiten der einzeln verpackten Hygieneartikel oder einer weiteren den Stapel umgebenden Verpackung. Diese auftretende Reibung kann zum Abrieb eines auf der Außenseite des Verpackungsmittels aufgebrachten Aufdrucks führen. Der Rückgriff auf die Bedruckung der Backsheetseite des absorbierenden Artikels selbst bringt indessen herstellungstechnische Schwierigkeiten mit sich und ist im Bereich der Haftmittelbeschichtung gerade nicht sinnvoll möglich. Außerdem wird hierdurch die Erkennbarkeit des absorbierenden Artikels in an sich nicht wünschenswerter Weise begünstigt.

Der vorliegenden Erfindung liegt die Aufgabe zugrunde, einen einzeln verpackten Hygieneartikel zu schaffen, welcher auf einfache und damit wirtschaftliche Weise betriebssicher herstellbar ist, bei dem das vorstehend geschilderte Problem der Beeinträchtigung einer aufgebrachten Dekoration besser beherrscht werden kann, ohne dass allzugroße Gestaltungseinschränkungen hingenommen werden müssen.

Diese Aufgabe wird erfindungsgemäß gelöst durch einen einzeln verpackten Hygieneartikel mit den Merkmalen des Anspruchs 1.

Es wird also eine auf ihrer Innenseite bedruckte Kunststofffilmschicht als Verpackungsmittel verwendet. Kunststofffilmschichten können in ihrer erwünschten Durchsichtigkeit und hinsichtlich des Grads ihrer diffusen Lichtstreuung weitgehend frei gewählt werden, so dass auch ein auf ihre Innenseite aufgebrachtes Kennzeichnungs- oder Dekorationsmittel durch die Kunststofffilmschicht hindurch, insbesondere auch bei Auflichtbeleuchtung, gut sichtbar, also visuell wahrnehmbar sein kann. Durch die Aufbringung des Kennzeichnungs- oder Dekorationsmittels auf das Verpackungsmittel selbst und nicht auf das Backsheet des absorbierenden Artikels bleibt dessen Gestaltung und gegebenenfalls zusätzliche Dekoration unbeeinflusst. Weiter wird erfindungsgemäß vorgeschlagen, dass im Überlappungsbereich mit der Haftmittelbeschichtung auf der Außenseite des Backsheets das Kennzeichnungs- oder Dekorationsmittel von einer als Schutzabdeckung wirkenden Schicht überdeckt und hierdurch vor Abrieb und Interaktion mit der Haftmittelbeschichtung geschützt ist. Auf diese Weise lässt sich eine vor äußeren und inneren Einflüssen geschützte Gestaltung des Verpackungsmittels mit einem visuell wahrnehmbaren Kennzeichnungs- oder Dekorationsmittel realisieren, und zwar auch, wenn der absorbierende Artikel eine Haftmittelbeschichtung zur lösbaren Anhaftung an ein Unterbekleidungsstück aufweist.

Erfindungsgemäß wird vorgeschlagen, dass die als Schutzabdeckung wirkende Schicht in Form einer zumindest bereichsweisen Beschichtung der nach innen gewandten Oberfläche der Kunststofffilmschicht ausgebildet ist, die unmittelbar und flächenhaft auf die nach innen gewandte Oberfläche der Kunststofffilmschicht aufgebracht ist, so dass das Kennzeichnungs- oder Dekorationsmittel sandwichartig zwischen der Kunststofffilmschicht und der Beschichtung angeordnet ist. Dies bringt den Vorteil mit sich, dass die das Kennzeichnungs- oder Dekorationsmittel schützende Beschichtung zugleich als Ablöseschicht für die Haftmittelbeschichtung auf dem Backsheet dienen kann, eine zusätzliche Ablöseschicht oder Lage hierfür also eingespart werden kann.

Weiter erweist es sich als vorteilhaft, wenn die zumindest bereichsweise Beschichtung der nach innen gewandten Oberfläche der Kunststofffilmschicht von einer vorzugsweise farblosen und vorzugsweise transparenten Beschichtungsmasse mit Antihafteigenschaften, insbesondere einer Beschichtungsmasse auf Silikonbasis, gebildet ist. Diese Beschichtung kann in an sich beliebiger Weise ausgebildet und aufgebracht sein. Es kann sich hierbei beispielsweise um eine weitere zuvor hergestellte und dann aufgebrachte Filmschicht handeln. Demgegenüber wird vorgeschlagen, dass es sich um eine im Zuge der Herstellung der Kunststofffilmschicht nach dem Aufbringen des Kennzeichnungs- oder Dekorationsmittels aufkaschierte Beschichtungsmasse mit den genannten Eigenschaften handelt.

Weiter wird vorgeschlagen, dass die zumindest bereichsweise Beschichtung der nach innen gewandten Oberfläche der Kunststofffilmschicht im gesamten Überlappungsbereich zwischen dem Kennzeichnungs- oder Dekorationsmittel und der Haftmittelbeschichtung auf der körperabgewandten Außenseite des Backsheets vorhanden ist und eine flächenhaft durchgehende Beschichtung bildet. Unter einer flächenhaft durchgehenden Beschichtung wird eine Beschichtung ohne Öffnungen, Löcher oder dergleichen unbeschichteten Bereichen verstanden. Solchenfalls ist es möglich, die Kunststofffilmschicht als Verpackungsmittel fertig vorzukonfektionieren.

Weiter kann es sich als vorteilhaft erweisen, wenn die zumindest bereichsweise Beschichtung die gesamte nach innen gewandte Oberfläche der Kunststofffilmschicht überfängt. Es wäre indessen auch denkbar und vorteilhaft, die Kunststofffilmschicht teilweise, insbesondere in Randbereichen, insbesondere entlang von Längsrändern, unbeschichtet zu belassen, so dass dort also nur das ursprüngliche Material der Kunststofffilmschicht vorhanden ist, beispielsweise um dort Siegelnähte anzubringen, die insbesondere durch Ultraschallfügetechnik hergestellt werden können oder als Heißsiegelnähte mit oder ohne Verwendung zusätzlicher Haftvermittlungsmaterialien hergestellt werden können.

Weiter erweist es sich als vorteilhaft, dass zwischen der bereichsweisen Beschichtung der nach innen gewandten Oberfläche der Kunststofffilmschicht und der Haftmittelbeschichtung des Backsheets keine weitere Schicht oder Lage vorhanden ist, so dass die bereichsweise Beschichtung unmittelbar gegen die Haftmittelbeschichtung des Backsheets anliegt. Auf diese Weise bildet die zumindest bereichsweise Beschichtung auf der nach innen gewandten Oberfläche der Kunststofffilmschicht, welche das dort aufgebrachte Kennzeichnungs- oder Dekorationsmittel überfängt und schützt, zugleich eine Abdeckung der Haftmittelbeschichtung auf dem Backsheet.

Es hat sich ferner als vorteilhaft erwiesen, wenn die Kunststofffilmschicht ein thermoplastisches Polymer, insbesondere aus der Klasse der Polyolefine, insbesondere Polypropylen und/oder Polyäthylen, umfasst oder daraus besteht. Dies schließt allerdings nicht aus, dass die Kunststofffilmschicht außenseitig eine dünne und durchsichtige Vliesbeschichtung aufweisen kann, um einen textilen Eindruck und ein haptisch angenehmes Gefühl zu vermitteln.

Weiter ist es denkbar und für viele Anwendungen vorteilhaft, wenn die Kunststofffilmschicht nichtpolymere Füllstoffe umfasst, wie insbesondere Pigmente, Titandioxid oder Carbonate, Weichmacher, Duftstoffe, antimikrobielle Substanzen. Insbesondere kann durch Einbringen körniger Substanzen und geringfügiges Recken der Kunststofffilmschicht in an sich bekannter Weise eine Mikroporosität und damit eine Atmungsaktivität in einem erwünschten Umfang realisiert werden.

Die das Verpackungsmittel bildende Kunststofffilmschicht soll hinreichend stabil sein, wobei ein ökonomischer Ressourceneinsatz gewählt werden soll. Es erweist sich insoweit als vorteilhaft, wenn die Kunststofffilmschicht ein Flächengewicht von mindestens 10 g/m² insbesondere von mindestens 12 g/m² und höchstens 35 g/m², insbesondere von höchstens 30 g/m², von insbesondere höchstens 28 g/m² und weiter insbesondere von höchstens 24 g/m² aufweist.

Es erweist sich weiter als vorteilhaft, wenn das Kennzeichnungs- oder Dekorationsmittel im Wesentlichen wasserunlöslich ist. Als wasserunlöslich wird das Kennzeichnungs- oder Dekorationsmittel bezeichnet und angesehen, wenn es in seiner auf die Kunststofffilmschicht aufgebrachten Form bei Kontakt mit Wasser oder Feuchtigkeit seine Form und Anmutung behält und nicht beispielsweise verläuft oder sich von der Kunststofffilmschicht löst. Im Bereich der Drucktechnik spricht man hier von sogenannter "Nassechtheit".

Es wird weiter vorgeschlagen, dass das Kennzeichnungs- oder Dekorationsmittel im Wege des Flexo-Drucks, Inkjet-Drucks oder Siebdrucks aufgebracht ist.

Dabei kann das Kennzeichnungs- oder Dekorationsmittel wenigstens einen Farbstoff oder ein Farbpigment umfassen oder daraus bestehen.

Es wird auch eine mehrfarbige Gestaltung als vorteilhaft erachtet. Insoweit wird vorgeschlagen, dass das Kennzeichnungs- oder Dekorationsmittel mindestens einen ersten Bereich einer ersten Farbe, insbesondere mindestens den ersten Bereich und einen zweiten Bereich einer von der ersten Farbe verschiedenen zweiten Farbe, weiter insbesondere mindestens den ersten Bereich und den zweiten Bereich und einen dritten Bereich einer von der ersten und der zweiten Farbe verschiedenen dritten Farbe umfasst.

Weiter wird vorgeschlagen, dass das Kennzeichnungs- oder Dekorationsmittel über wenigstens 5 %, insbesondere wenigstens 8 %, insbesondere wenigstens 10 %, insbesondere wenigstens 15 %, insbesondere wenigstens 20 %, insbesondere wenigstens 30 % und über höchstens 100 %, insbesondere bis höchstens 90 %, insbesondere bis höchstens 80 %, insbesondere bis höchstens 70 %, insbesondere bis höchstens 60 %, insbesondere bis höchstens 50 % einer Gesamtfläche der nach innen gewandten Oberfläche der Kunststofffilmschicht erstreckt ist.

Bei dem Kennzeichnungs- oder Dekorationsmittel handelt es sich um eine beliebige optisch und visuell wahrnehmbare Information im weitesten Sinn. Insbesondere kann es sich als vorteilhaft erweisen, wenn das Kennzeichnungs- oder Dekorationsmittel eine zusammenhängende Struktur, insbesondere in Form eines sich periodisch wiederholenden Musters, bildet und/oder voneinander getrennte Elemente, welche vollumfänglich durch unbedruckte Bereiche der nach innen gewandten Oberfläche der Kunststofffilmschicht umgeben sind, aufweist oder jeweils hieraus besteht.

Weiter wäre es denkbar und für eine Informationsübermittlung vorteilhaft, wenn das Kennzeichnungs- oder Dekorationsmittel Schriftzeichen umfasst, die spiegelverkehrt auf die nach innen gewandte Oberseite der Kunststofffilmschicht aufgedruckt sind, so dass sie bei Ansicht des verpackten Hygieneartikels von außen durch die Kunststofffilmschicht hindurch in Positivschrift visuell erfassbar sind.

Weiter kann das Kennzeichnungs- oder Dekorationsmittel eine maschinenlesbare Information, insbesondere Strichcode oder Matrixcode, umfassen.

Der hier in Rede stehende absorbierende Artikel kann ausgewählt sein aus der Gruppe von absorbierenden Einlagen, Vorlagen, Damenhygienebinden, Krankenunterlagen, Windeln des offenen oder geschlossenen Typs, wobei der absorbierende Artikel eine zwischen dem Topsheet und dem Absorptionskörper angeordnete Flüssigkeitsaufnahme- und/oder -verteilerlage umfassen kann.

Auch weitergehende Funktionsschichten oder an sich bekannte Ausstattungselemente von modernen absorbierenden Artikeln, wie sich emporerhebende seitliche Auslaufsperren oder weitere Chassis- oder Absorptionskörperelemente, können weiter vorgesehen sein.

Weiter kann es denkbar sein, dass der absorbierende Artikel zumindest bereichsweise lösbar mit der Kunststofffilmschicht des Verpackungsmittels unter Vermittlung einer Haftverbindung, insbesondere mittels eines Klebermaterials, verbunden ist.

Weiter ist es denkbar und vorteilhaft, dass der absorbierende Artikel flächenhaft auf die

Kunststofffilmschicht aufgelegt ist und an mindestens einer, insbesondere mindestens zwei, weiter insbesondere genau zwei Faltlinien zusammen mit der Kunststofffilmschicht auf sich selbst gefaltet ist, derart, dass eine gesamte äußere Oberfläche des Hygieneartikels von dem Verpackungsmittel gebildet ist.

Hierbei erweist es sich als vorteilhaft, wenn der absorbierende Artikel und dessen Verpackungsmittel derart gefaltet sind, dass das auf die nach innen gewandte Oberfläche der Kunststofffilmschicht aufgebrachte Kennzeichnungs- oder Dekorationsmittel nicht in direkten Kontakt mit dem Topsheet des absorbierenden Artikels gelangt. Auf diese Weise wird auch verhindert, dass ein Farbübertrag oder eine Beeinträchtigung des Kennzeichnungs- oder Dekorationsmittels durch Reibung mit dem Topsheet des absorbierenden Artikels erfolgt.

Weiter erweist es sich als vorteilhaft, wenn eine äußere Oberfläche des Verpackungsmittels kein Kennzeichnungs- oder Dekorationsmittel aufweist.

Wie schon angedeutet, kann es sich als vorteilhaft erweisen, wenn das Verpackungsmittel an zwei seitlichen Längsrandbereichen auf sich selbst gefügt ist, insbesondere mittels einer Siegelnaht oder Klebenaht, so dass durch das Verpackungsmittel ein zumindest entlang von Längsrändern geschlossener Aufnahmeraum für den darin aufgenommenen absorbierenden Artikel gebildet ist. Typischerweise bleibt ein Querrand zum Eingreifen mittels der Finger eines Benutzers ungefügt, was aber nur eine Ausführungsform darstellt.

Es ist auch denkbar und vorteilhaft, wenn das Verpackungsmittel ein Verschlussmittel, insbesondere einen klebend oder mechanisch haftenden Verschlussstreifen umfasst, mittels dessen eine gefaltete Konfiguration aus absorbierendem Artikel und Verpackungsmittel lösbar fixierbar ist und damit außerdem eine Wiederverwendung des Verpackungsmittels als Entsorgungspackung für den gebrauchten absorbierenden Artikel denkbar ist. Typischerweise ist dieser Verschlussstreifen im Bereich eines nicht gefügten Querrandbereichs des Verpackungsmittels vorgesehen.

Weitere Merkmale, Einzelheiten und Vorteile der Erfindung ergeben sich aus den beigefügten Patentansprüchen und aus der zeichnerischen Darstellung und nachfolgenden Beschreibung von Ausführungsformen der vorliegenden Erfindung. In der Zeichnung zeigt:
- Figur 1: eine Draufsicht auf einen absorbierenden Artikel, der auf ein Verpackungsmittel aufgelegt ist nach einer ersten Ausführungsform der Erfindung;
- Figur 2: eine schematische Schnittansicht mit Schnittebene I-I in Figur 1;
- Figur 3: eine Draufsicht auf einen absorbierenden Artikel, der auf ein Verpackungsmittel aufgelegt ist nach einer zweiten Ausführungsform der Erfindung;
- Figur 4: eine schematische Schnittansicht mit Schnittebene II-II in Figur 3;
- Figur 5: eine perspektivische Ansicht eines erfindungsgemäßen einzeln verpackten Hygieneartikels erhalten durch zweimalige Faltung des absorbierenden Artikels und des Verpackungsmittels der Figuren 1 und 2 bzw. 3 und 4 und Verschließen von Längsseitenrändern; und
- Figur 6: eine Draufsicht auf den einzeln verpackten Hygieneartikel nach Figur 5
.

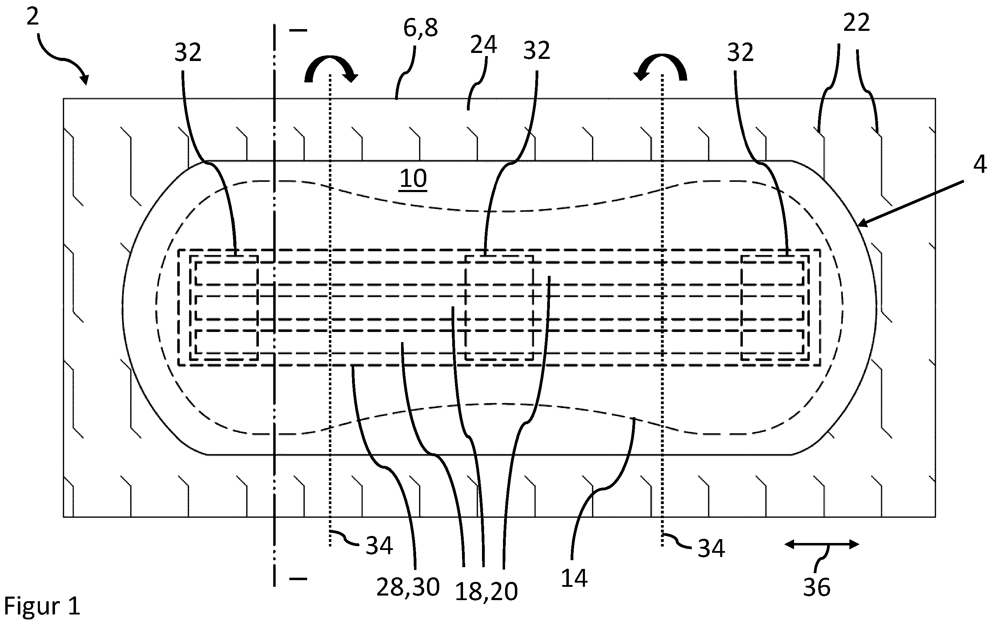

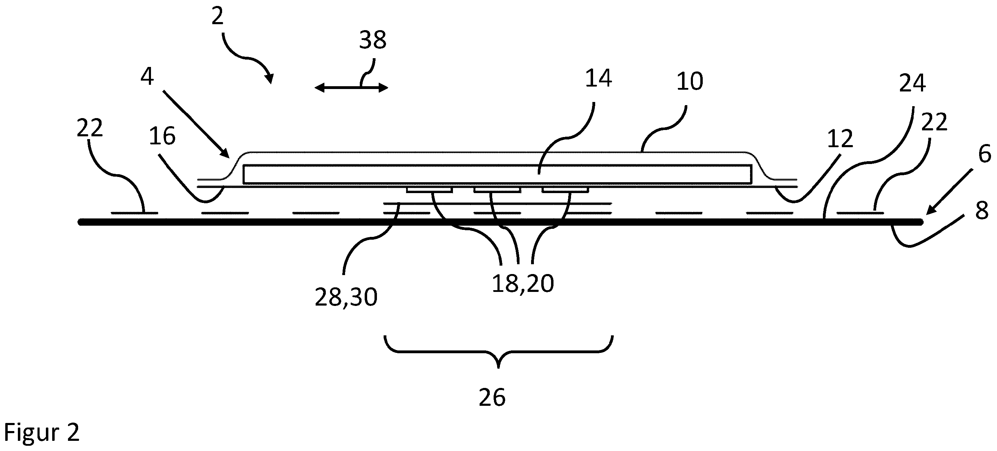

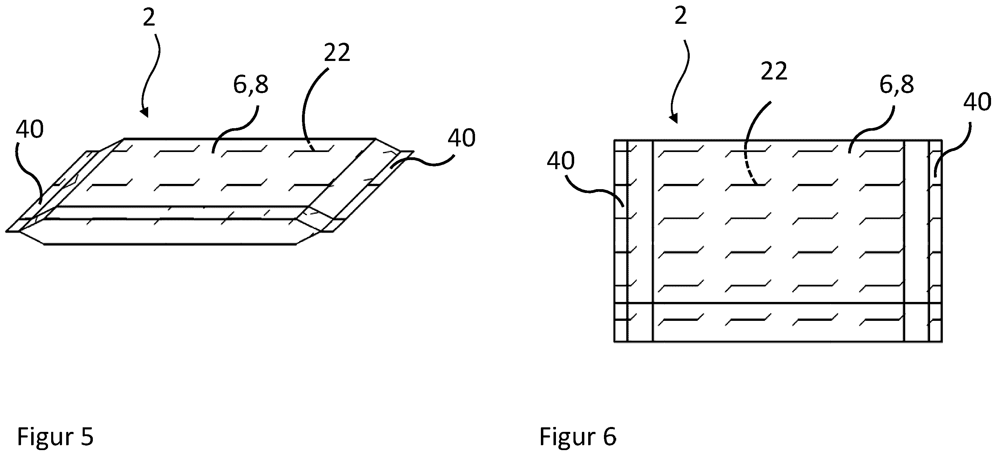

Die Figuren 1 und 2 zeigen in teilweise schematisierender Darstellung die Komponenten zur Bildung eines erfindungsgemäßen einzeln verpackten Hygieneartikels 2, der in Figuren 5 und 6 im gefalteten und fertig verpackten Zustand dargestellt ist. Diese Komponenten umfassen einen absorbierenden Artikel 4, der hier beispielhaft in Form einer Damenbinde, Slipeinlage oder Inkontinenzvorlage dargestellt ist, sowie ein Verpackungsmittel 6 in Form eines zunächst ebenen Abschnitts einer Kunststofffilmschicht 8. Der absorbierende Artikel 4 ist im eben ausgebreiteten Zustand auf die ebenfalls eben ausgebreitete Kunststofffilmschicht 8 aufgelegt.

Der absorbierende Artikel 4 umfasst ein körperzugewandtes flüssigkeitsdurchlässiges Topsheet 10, gegebenenfalls eine daran anschließende Flüssigkeitsaufnahme- und - verteilerschicht, und ein flüssigkeitsundurchlässiges Backsheet 12 und einen dazwischen angeordneten Absorptionskörper 14. Auf einer körperabgewandten Außenseite 16 des Backsheets 12 ist eine Haftmittelbeschichtung 18 zur lösbaren Anhaftung des absorbierenden Artikel 4 an einem Bekleidungsstück vorgesehen. Die Haftmittelbeschichtung 18 ist beispielhaft in Form von drei parallelen Kleberstreifen 20 ausgebildet.

Das von der Kunststofffilmschicht 8 gebildete Verpackungsmittel 6 umfasst ein visuell wahrnehmbares Kennzeichnungs- oder Dekorationsmittel 22. Erfindungsgemäß ist dieses Kennzeichnungs- oder Dekorationsmittel 22 auf eine nach innen gewandte Oberfläche 24 der Kunststofffilmschicht 8 aufgedruckt. Zumindest in diesem Bereich ist die Kunststofffilmschicht 8 derart durchsichtig ausgebildet, dass das Kennzeichnungs- oder Dekorationsmittel 22 von außen durch die Kunststofffilmschicht 8 hindurch visuell wahrnehmbar ist. In einem Überlappungsbereich 26 des Kennzeichnungs- oder Dekorationsmittels 22 mit der Haftmittelbeschichtung 18 auf der körperabgewandten Außenseite 16 des Backsheets 12 ist eine als Schutzabdeckung wirkende Schicht 28 zwischen dem Kennzeichnungs- oder Dekorationsmittel 22 und der Haftmittelbeschichtung 18 vorhanden. Diese als Schutzabdeckung wirkende Schicht 28 überfängt in diesem Überlappungsbereich 26 sowohl das Kennzeichnungs- oder Dekorationsmittel 22 vollständig als auch vorzugsweise die gesamte Haftmittelbeschichtung 18. Auf diese Weise wird ein direkter unmittelbarer Kontakt zwischen der Haftmittelbeschichtung 18 und dem Kennzeichnungs- oder Dekorationsmittel 22 verhindert. Eine Beeinträchtigung des Kennzeichnungs- oder Dekorationsmittels 22 durch einen solchen Kontakt wird daher vermieden. Insbesondere lässt sich hierdurch vermeiden, dass das Kennzeichnungs- oder Dekorationsmittel 22 durch die Haftmittelbeschichtung abgelöst, abgetragen oder in sonstiger Weise beeinträchtigt, insbesondere verwischt wird. Zugleich aber vermag diese als Schutzabdeckung wirkende Schicht 28 auch zur Deaktivierung der Haftmittelbeschichtung 18 bis zur tatsächlichen Ingebrauchnahme und Anhaftung an ein Unterbekleidungsstück zu dienen. Im beispielhaft dargestellten Fall dieser Ausführungsform ist diese Schicht 28 von einem Flachmaterialabschnitt 30, vorzugsweise mit Antihafteigenschaften zumindest auf der der Haftmittelbeschichtung 18 zugewandten Seite, insbesondere mit einer Antihaftbeschichtung, gebildet. Dem Flachmaterialabschnitt 30 kommt hier also eine Doppelfunktion zu; er stützt das Kennzeichnungs- oder Dekorationsmittel 22 und wirkt als "Releaseliner", also ablösbare Abdeckung, für die Haftmittelbeschichtung 18. Wie eingangs erwähnt, kann der Flachmaterialabschnitt 30 im Zuge der Herstellung zuerst an den absorbierenden Artikel 4 angebracht und dann mit diesem auf die Kunststofffilmschicht 8 aufgelegt werden, oder der Flachmaterialabschnitt 30 könnte zuerst auf die Kunststofffilmschicht 8 aufgebracht werden. Insbesondere ist es denkbar und vorteilhaft, wenn der Flachmaterialabschnitt über eine in Figur 1 nur schematisch angedeutete Fügeverbindung 32 an die nach innen gewandte Oberfläche 24 der Kunststofffilmschicht 8 angefügt ist, so dass er beim Abnehmen des absorbierenden Artikels 4 von der Kunststofffilmschicht 8 an der Kunststofffilmschicht 8 verbleibt.

In Figur 1 sind ferner zwei Faltlinien 34 angedeutet, um die der absorbierende Artikel 4 samt der Kunststofffilmschicht 8 auf sich selbst gefaltet wird. Auf diese Weise entsteht eine gefaltete Konfiguration, wie sie in Figuren 5 und 6 dargestellt ist. Die Faltlinien 34 verlaufen dabei orthogonal zu einer in Figur 1 mit Bezugszeichen 36 dargestellten Längsrichtung des absorbierenden Artikels 4, also in einer Querrichtung 38. In Längsrichtung 36 verlaufende Längsrandbereiche 40 des gefalteten Verpackungsmittels 6, also der Kunststofffilmschicht 8, sind dabei gegeneinander gefügt, beispielsweise auf die eine oder andere eingangs erwähnte Weise.

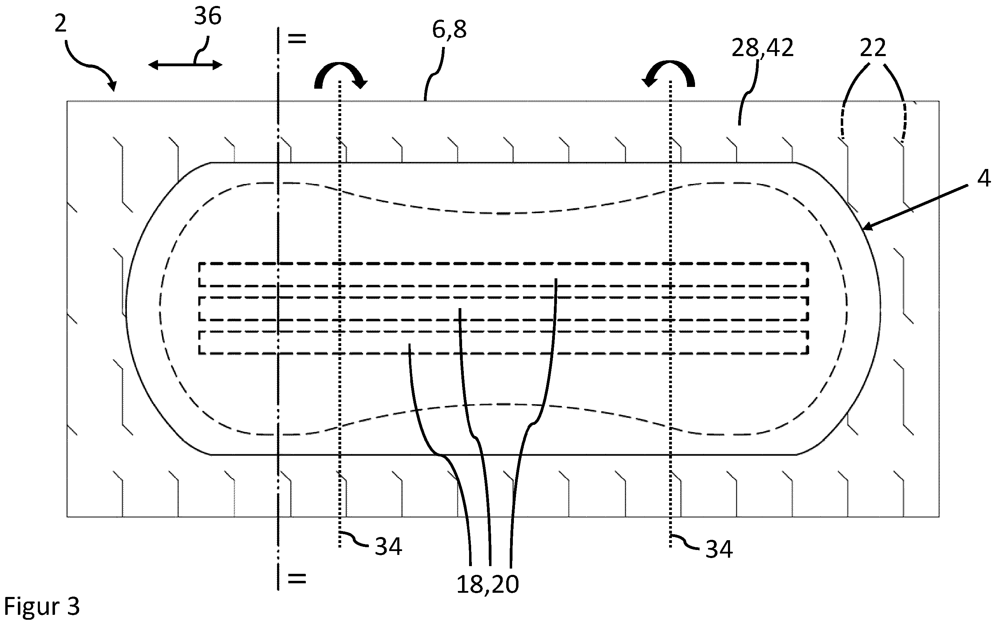

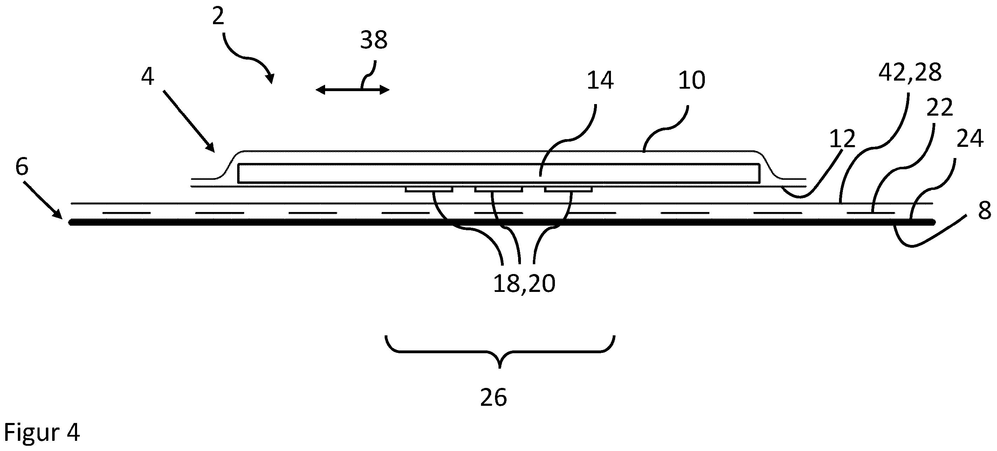

Die in Figuren 3 und 4 dargestellte weitere Ausführungsform des einzeln verpackten Hygieneartikels 2 unterscheidet sich von der vorausgehend beschriebenen Ausführungsform dadurch, dass anstelle des Flachmaterialabschnitts 30 zur Bildung einer als Schutzabdeckung des Kennzeichnungs- oder Dekorationsmittels 22 wirkenden Schicht 28 eine hier beispielhaft durchgehende Beschichtung 42 der nach innen gewandten Oberfläche 24 der Kunststofffilmschicht 8 ausgebildet ist. Es wäre denkbar, dass diese Beschichtung 42 nur bereichsweise im Überlappungsbereich 26 mit der Haftmittelbeschichtung 18 ausgebildet ist. Sie ist aber im beispielhaft dargestellten Fall über die gesamte nach innen gewandte Oberfläche 24 der Kunststofffilmschicht 8 aufgebracht, so dass das Kennzeichnungs- oder Dekorationsmittel 22 auf der Kunststofffilmschicht 8 vollständig durch die Beschichtung 42 als Schutzabdeckung wirkende Schicht 28 überfangen und sandwichartig zwischen der Kunststofffilmschicht 8 und dieser Beschichtung 42 aufgenommen ist. Die Beschichtung 42, das Kennzeichnungs- und Dekorationsmittel 22 und die Kunststofffilmschicht 8 bilden also eine Verbundschicht. Die Beschichtung 42 kann optional auch derart ausgebildet sein, dass die in Figuren 5 und 6 dargestellten in Längsrichtung verlaufenden und gegeneinander gefügten Längsrandbereiche 40 des gefalteten Verpackungsmittels, frei von der Beschichtung sind. Die Beschichtung 42 kann wie eingangs ausführlich dargelegt beschaffen sein und weist vorzugsweise Antihafteigenschaften zumindest auf ihrer dem Backsheet 12 bzw. der Haftmittelbeschichtung 18 zugewandten Seite auf.

Die Beschichtung 42 dient also sowohl als Schutzabdeckung des Kennzeichnungs- oder Dekorationsmittels 22 als auch zugleich als Ablöseschicht für die Haftmittelbeschichtung 18. Wie man erkennt, ist keine zusätzliche Ablöseschicht/Releaseliner verwendet. Die Faltung von absorbierendem Artikel 4 und Verpackungsmittel 6 erfolgt wie bei der ersten Ausführungsform.

## Patentansprüche

1. Einzeln verpackter Hygieneartikel (2), umfassend einen absorbierenden Artikel (4) und ein den Artikel (4) vorzugsweise vollständig umschließendes Verpackungsmittel (6), wobei der absorbierende Artikel (4) zumindest ein körperzugewandtes Topsheet (10), ein körperabgewandtes Backsheet (12) und einen dazwischen angeordneten Absorptionskörper (14) aufweist, wobei eine körperabgewandte Außenseite (16) des Backsheets (12) bereichsweise eine Haftmittelbeschichtung (18) zur lösbaren Anhaftung des absorbierenden Artikels (4) an einem Bekleidungsstück aufweist und wobei die körperabgewandte Außenseite (16) des Backsheets (12) einer nach innen gewandten Oberfläche (24) des Verpackungsmittels (6) zugewandt ist, wobei das Verpackungsmittel (6) von einer Kunststofffilmschicht (8) gebildet ist, die ein visuell wahrnehmbares Kennzeichnungs- oder Dekorationsmittel (22) aufweist, wobei das visuell wahrnehmbare Kennzeichnungs- oder Dekorationsmittel (22) auf die nach innen gewandte Oberfläche (24) der Kunststofffilmschicht (8) aufgedruckt ist und die Kunststofffilmschicht (8) zumindest in diesem Bereich derart durchsichtig ausgebildet ist, dass das Kennzeichnungs- oder Dekorationsmittel (22) auch von außen durch die Kunststofffilmschicht (8) hindurch visuell wahrnehmbar ist, und wobei das Kennzeichnungs- oder Dekorationsmittel (22) zumindest in einem Überlappungsbereich (26) mit der Haftmittelbeschichtung (18) von einer als Schutzabdeckung wirkenden Schicht (28) zwischen der Haftmittelbeschichtung (18) und dem Kennzeichnungs- oder Dekorationsmittel (22) überfangen ist, **dadurch gekennzeichnet,**
**dass** die als Schutzabdeckung wirkende Schicht (28) in Form einer zumindest bereichsweisen Beschichtung (42) der nach innen gewandten Oberfläche (24) der Kunststofffilmschicht (8) ausgebildet ist, die unmittelbar und flächenhaft auf die nach innen gewandte Oberfläche (24) der Kunststofffilmschicht (8) aufgebracht ist, so dass das Kennzeichnungs- oder Dekorationsmittel (22) sandwichartig zwischen der Kunststofffilmschicht (8) und der Beschichtung (42) angeordnet ist, und
**dass** die zumindest bereichsweise Beschichtung (42) der nach innen gewandten Oberfläche (24) der Kunststofffilmschicht (8) von einer Beschichtungsmasse mit Antihafteigenschaften gebildet ist, und
**dass** die zumindest bereichsweise Beschichtung (42) der nach innen gewandten Oberfläche (24) der Kunststofffilmschicht (8) im gesamten Überlappungsbereich (26) zwischen dem Kennzeichnungs- oder Dekorationsmittel (22) und der Haftmittelbeschichtung (18) auf der körperabgewandten Außenseite (16) des Backsheets (12) vorhanden ist und eine flächenhaft durchgehende Beschichtung bildet, und dass zwischen der Beschichtung (42) der nach innen gewandten Oberfläche (24) der Kunststofffilmschicht (8) und der Haftmittelbeschichtung (18) des Backsheets (12) keine weitere Schicht oder Lage vorhanden ist, so dass die bereichsweise Beschichtung (42) unmittelbar gegen die Haftmittelbeschichtung (18) des Backsheets (12) anliegt.

2. Einzeln verpackter Hygieneartikel (2) nach Anspruch 1, **dadurch gekennzeichnet, dass** die zumindest bereichsweise Beschichtung (42) der nach innen gewandten Oberfläche (24) der Kunststofffilmschicht (8) von einer farblosen und transparenten Beschichtungsmasse mit Antihafteigenschaften, insbesondere einer Beschichtungsmasse auf Silikonbasis, gebildet ist.

3. Einzeln verpackter Hygieneartikel (2) nach Anspruch 1 oder 2, **dadurch gekennzeichnet, dass** die zumindest bereichsweise Beschichtung (42) die gesamte nach innen gewandte Oberfläche (24) der Kunststofffilmschicht (8) überfängt.

4. Einzeln verpackter Hygieneartikel (2) nach einem oder mehreren der vorstehenden Ansprüche **dadurch gekennzeichnet, dass** die Kunststofffilmschicht (8) ein thermoplastisches Polymer, insbesondere aus der Klasse der Polyolefine, insbesondere Polypropylen und/oder Polyäthylen, umfasst oder daraus besteht.

5. Einzeln verpackter Hygieneartikel (2) nach einem oder mehreren der vorstehenden Ansprüche **dadurch gekennzeichnet, dass** die Kunststofffilmschicht (8) ein Flächengewicht von mindestens 10 g/m² insbesondere von mindestens 12 g/m² und höchstens 35 g/m², insbesondere von höchstens 30 g/m², von insbesondere höchstens 28 g/m² und weiter insbesondere von höchstens 24 g/m² aufweist.

6. Einzeln verpackter Hygieneartikel (2) nach einem oder mehreren der vorstehenden Ansprüche **dadurch gekennzeichnet, dass** das Kennzeichnungs- oder Dekorationsmittel (22) im wesentlichen wasserunlöslich ist.

7. Einzeln verpackter Hygieneartikel (2) nach einem oder mehreren der vorstehenden Ansprüche **dadurch gekennzeichnet, dass** der absorbierende Artikel (4) und dessen Verpackungsmittel (6) derart gefaltet sind, dass das auf die nach innen gewandte Oberfläche (24) der Kunststofffilmschicht (8) aufgebrachte Kennzeichnungs- oder Dekorationsmittel (22) nicht in direkten Kontakt mit dem Topsheet (10) des absorbierenden Artikels (4) gelangt.

## Claims

1. Individually packaged sanitary article (2), comprising an absorbent article (4) and a packaging means (6) which preferably completely encloses the article (4), the absorbent article (4) having at least one top sheet (10) facing the body, a back sheet (12) facing away from the body, and an absorbent body (14) arranged therebetween, an outer side (16) of the back sheet (12) facing away from the body having, in regions, an adhesive coating (18) for releasably adhering the absorbent article (4) to a garment, and the outer side (16) of the back sheet (12) facing away from the body facing an inward-facing surface (24) of the packaging means (6), the packaging means (6) being formed by a plastics material film layer (8) which has a visually perceptible marking or decoration means (22), the visually perceptible marking or decoration means (22) being printed on the inward-facing surface (24) of the plastics material film layer (8), and the plastics material film layer (8) being designed to be transparent, at least in this region, such that the marking or decoration means (22) is also visually perceptible from the outside through the plastics material film layer (8), and the marking or decoration means (22) being covered, at least in an overlapping region (26) with the adhesive coating (18), by a layer (28) acting as a protective cover between the adhesive coating (18) and the marking or decoration means (22), **characterized in that**
the layer (28) acting as a protective cover is designed in the form of a coating (42) of the inward-facing surface (24) of the plastics material film layer (8), at least in regions, which layer is applied directly and in a planar manner to the inward-facing surface (24) of the plastics material film layer (8) such that the marking or decoration means (22) is arranged in a sandwich-like manner between the plastics material film layer (8) and the coating (42), and
**in that** the coating (42) of the inward-facing surface (24) of the plastics material film layer (8), at least in regions, is formed by a coating composition which has anti-adhering properties, and
**in that** the coating (42) of the inward-facing surface (24) of the plastics material film layer (8), at least in regions, is present in the entire overlapping region (26) between the marking or decoration means (22) and the adhesive coating (18) on the outer side (16) of the back sheet (12) facing away from the body, and said coating forms a planar, continuous coating, and
**in that**, between the coating (42) of the inward-facing surface (24) of the plastics material film layer (8) and the adhesive coating (18) of the back sheet (12), there is no additional layer or ply, such that, in regions, the coating (42) directly abuts the adhesive coating (18) of the back sheet (12).

2. Individually packaged sanitary article (2) according to claim 1, **characterized in that** the coating (42) of the inward-facing surface (24) of the plastics material film layer (8), at least in regions, is formed by a colorless and transparent coating composition which has anti-adhering properties, in particular by a silicone-based coating composition.

3. Individually packaged sanitary article (2) according to claim 1 or 2, **characterized in that** the coating (42) covers the entire inward-facing surface (24) of the plastics material film layer (8), at least in regions.

4. Individually packaged sanitary article (2) according to one or more of the preceding claims, **characterized in that** the plastics material film layer (8) comprises or consists of a thermoplastic polymer, in particular from the class of polyolefins, in particular polypropylene and/or polyethylene.

5. Individually packaged sanitary article (2) according to one or more of the preceding claims, **characterized in that** the plastics material film layer (8) has an area density of at least 10 g/m², in particular at least 12 g/m², and at most 35 g/m², in particular at most 30 g/m², in particular at most 28 g/m², and more particularly at most 24 g/m².

6. Individually packaged sanitary article (2) according to one or more of the preceding claims, **characterized in that** the marking or decoration means (22) is substantially water-insoluble.

7. Individually packaged sanitary article (2) according to one or more of the preceding claims, **characterized in that** the absorbent article (4) and its packaging means (6) are folded such that the marking or decoration means (22) applied to the inward-facing surface (24) of the plastics material film layer (8) does not come into direct contact with the top sheet (10) of the absorbent article (4).

## Revendications

1. Article d'hygiène (2) emballé individuellement, comprenant un article absorbant (4) et un moyen d'emballage (6) enveloppant de préférence complètement ledit article (4), dans lequel l'article absorbant (4) comprend au moins une feuille supérieure (10) montrant dans la direction du corps, une feuille arrière (12) montrant dans la direction opposée au corps et un corps d'adsorption (14) disposé entre celles-ci, dans lequel une face extérieure (16) de la feuille arrière (12), qui montre dans la direction opposée au corps, est munie par zones d'un revêtement d'adhésif (18) permettant le collage amovible de l'article absorbant (4) sur un vêtement, et dans lequel la face extérieure (16) de la feuille arrière (12), qui montre dans la direction opposée au corps, montre vers une surface (24) du moyen d'emballage (6), qui montre vers l'intérieur, dans lequel le moyen d'emballage (6) est constitué d'une couche en film plastique (8) qui présente un élément de marquage ou de décoration visuellement perceptible (22),
dans lequel l'élément de marquage ou de décoration visuellement perceptible (22) est imprimé sur la surface (24) de la couche en film plastique (8), qui montre vers l'intérieur, et ladite couche en film plastique (8) est conçue, au moins dans cette zone, de manière transparent de telle sorte que l'élément de marquage ou de décoration (22) soit également perceptible visuellement de l'extérieur à travers la couche en film plastique (8), et dans lequel l'élément de marquage ou de décoration (22) est recouvert, au moins dans une zone de chevauchement (26) avec le revêtement d'adhésif (18), par une couche (28) faisant office de couverture protectrice entre le revêtement d'adhésif (18) et l'élément de marquage ou de décoration (22), **caractérisé par le fait**
**que** la couche (28) faisant office de couverture protectrice est conçue sous la forme d'un revêtement présent au moins par zones (42) de la surface (24) de la couche en film plastique (8), qui montre vers l'intérieur, lequel revêtement (42) est appliqué directement et en nappe sur la surface (24) de la couche en film plastique (8), montrant vers l'intérieur, de sorte que l'élément de marquage ou de décoration (22) est disposé de type sandwich entre la couche en film plastique (8) et le revêtement (42), et
**que** le revêtement présent au moins par zones (42) de la surface (24) de la couche en film plastique (8), qui montre vers l'intérieur, est formé par une masse de revêtement ayant des propriétés antiadhésives, et
**que** le revêtement présent au moins par zones (42) de la surface (24) de la couche en film plastique (8), qui montre vers l'intérieur, est présent dans toute la zone de chevauchement (26) entre l'élément de marquage ou de décoration (22) et le revêtement d'adhésif (18) sur la face extérieure (16) de la feuille arrière (12), qui montre dans la direction opposée au corps, et forme un revêtement continu en nappe, et
et **qu'**aucune autre couche n'est présente entre le revêtement (42) de la surface (24) de la couche en film plastique (8), qui montre vers l'intérieur, et le revêtement d'adhésif (18) de la feuille arrière (12), de sorte que le revêtement présent par zones (42) est directement en appui contre le revêtement d'adhésif (18) de la feuille arrière (12).

2. Article d'hygiène emballé individuellement (2) selon la revendication 1, **caractérisé par le fait que** le revêtement présent au moins par zones (42) de la surface (24) de la couche en film plastique (8), qui montre vers l'intérieur, est constitué d'une masse de revêtement incolore et transparente ayant des propriétés antiadhésives, en particulier d'une masse de revêtement à base de silicone.

3. Article d'hygiène emballé individuellement (2) selon la revendication 1 ou 2, **caractérisé par le fait que** le revêtement présent au moins par zones (42) recouvre la totalité de la surface (24) de la couche en film plastique (8), qui montre vers l'intérieur.

4. Article d'hygiène emballé individuellement (2) selon une ou plusieurs des revendications précédentes, **caractérisé par le fait que** la couche en film plastique (8) comprend ou est constitué d'un polymère thermoplastique, en particulier de la classe des polyoléfines, en particulier de polypropylène et/ou de polyéthylène.

5. Article d'hygiène emballé individuellement (2) selon une ou plusieurs des revendications précédentes, **caractérisé par le fait que** la couche en film plastique (8) présente un grammage d'au moins 10 g/m², en particulier d'au moins 12 g/m² et de 35 g/m² tout au plus, en particulier de 30 g/m² tout au plus, en particulier de 28 g/m² tout au plus et en outre en particulier de 24 g/m² tout au plus.

6. Article d'hygiène emballé individuellement (2) selon une ou plusieurs des revendications précédentes, **caractérisé par le fait que** ledit élément de marquage ou de décoration (22) est pour l'essentiel insoluble dans l'eau.

7. Article d'hygiène emballé individuellement (2) selon une ou plusieurs des revendications précédentes, **caractérisé par le fait que** l'article absorbant (4) et son moyen d'emballage (6) sont pliés de telle sorte que l'élément de marquage ou de décoration (22) appliqué à la surface (24) de la couche en film plastique (8), qui montre vers l'intérieur, n'entre pas en contact direct avec la feuille supérieure (10) de l'article absorbant (4).
